(19) Europäisches Patentamt
European Patent Office
Office européen des brevets

(11) **EP 2 292 145 A1**

(12) **EUROPEAN PATENT APPLICATION**
published in accordance with Art. 153(4) EPC

(43) Date of publication:
**09.03.2011 Bulletin 2011/10**

(51) Int Cl.:
*A61B 5/1455* (2006.01)  *G01N 21/35* (2006.01)

(21) Application number: **09770117.1**

(22) Date of filing: **22.06.2009**

(86) International application number:
**PCT/JP2009/061315**

(87) International publication number:
**WO 2009/157407 (30.12.2009 Gazette 2009/53)**

(84) Designated Contracting States:
**AT BE BG CH CY CZ DE DK EE ES FI FR GB GR HR HU IE IS IT LI LT LU LV MC MK MT NL NO PL PT RO SE SI SK TR**
Designated Extension States:
**AL BA RS**

(30) Priority: **24.06.2008 JP 2008164482**

(71) Applicants:
- **Kabushiki Kaisha Toyota Jidoshokki**
  **Kariya-shi**
  **Aichi-ken**
  **Aichi 448-8671 (JP)**
- **Kyushu University,**
  **National University Corporation**
  **Higashi-ku**
  **Fukuoka-shi**
  **Fukuoka 812-8581 (JP)**

(72) Inventors:
- **MAKI, Takeshi**
  **Kariya-shi**
  **Aichi 448-8671 (JP)**

- **SHIMO, Toshihisa**
  **Kariya-shi**
  **Aichi 448-8671 (JP)**
- **KUMAGAI, Kyoko**
  **Kariya-shi**
  **Aichi 448-8671 (JP)**
- **HAYASHI, Hidetaka**
  **Kariya-shi**
  **Aichi 448-8671 (JP)**
- **YANAGISAWA, Hideo**
  **Kariya-shi**
  **Aichi 448-8671 (JP)**
- **TSUTSUI, Yusuke**
  **Kariya-shi**
  **Aichi 448-8671 (JP)**
- **SAWADA, Renshi**
  **Fukuoka-shi**
  **Fukuoka 812-8581 (JP)**
- **IWASAKI, Wataru**
  **Fukuoka-shi**
  **Fukuoka 812-8581 (JP)**

(74) Representative: **TBK-Patent**
**Bavariaring 4-6**
**80336 München (DE)**

(54) **NONINVASIVE ALCOHOL SENSOR**

(57) The present invention provides a noninvasive alcohol sensor that measures ethanol concentration with high accuracy by suppressing error dependent on glucose concentration. An alcohol sensor 10 includes first light-emitting means 21, second light-emitting means 22 and third light-emitting means 23. The first light-emitting means 21 radiates light having a wavelength of 1185 nm. This wavelength of 1185 nm is a wavelength at which the light absorbance of water and the light absorbance of ethanol are equal. The second light-emitting means 22 radiates light having a wavelength of 1710 nm. The third light-emitting means 23 radiates light having a wavelength of 1750 nm. Light detection means 30 detects the intensity of each light, and control means 60 calculates ethanol concentration based on the detected light intensity.

FIG. 1

**Description**

TECHNICAL FIELD

The present invention relates to a noninvasive alcohol sensor.

BACKGROUND ART

Noninvasive analyzers that use an optical sensor are known as devices for measuring components contained in blood. For example, a blood analyzer described in Patent Document 1 is provided with at least three light-emitting elements that radiate a body with different wavelengths of light, and a light-receiving element arranged at a position opposite to them with respect to the body. When the light-receiving element receives light that has passed through the body and generates photoelectric current in response to this, the concentration of a component in the blood is calculated based on the photoelectric current.

This type of analyzer can also be applied as an alcohol sensor for detecting ethanol concentration in the blood.

Patent Document 1: Japanese Patent Application Laid-open No. 2004-248820

DISCLOSURE OF THE INVENTION

PROBLEMS TO BE SOLVED BY THE INVENTION

However, since conventional alcohol sensors produce error dependent on glucose concentration in the measured ethanol concentration, there is a problem that ethanol concentration cannot be measured with high accuracy.

This is due to the reasons indicated below. Noninvasive alcohol sensors measure ethanol concentration by measuring the intensity of light absorbed by ethanol during the time light passes through the portion of the body being measured. However, since the body also contains glucose and this glucose also absorbs light, it is not possible to clearly distinguish between absorption attributable to ethanol and absorption attributable to glucose. Consequently, measurement errors occur due to the presence of glucose.

In order to solve such problems, an object of the present invention is to provide a noninvasive alcohol sensor that measures ethanol concentration with high accuracy by suppressing error dependent on the concentration of glucose.

MEANS FOR SOLVING THE PROBLEMS

In order to solve the above-mentioned problems, a noninvasive alcohol sensor related to the present invention is a noninvasive alcohol sensor that measures a concentration of ethanol contained in a specimen, comprising: first light-emitting means for radiating a first light having a first wavelength towards the specimen, second light-emitting means for radiating a second light having a second wavelength towards the specimen, third light-emitting means for radiating a third light having a third wavelength towards the specimen, light detection means for detecting light intensity, and calculation means for calculating the concentration of ethanol contained in the specimen based on intensity of the first light, intensity of the second light and intensity of the third light detected by the light detection means, wherein the first wavelength is a wavelength at which the light absorbance of water and the light absorbance of ethanol are equal.

Here, the light may be, for example, near-infrared light, and is not limited to visible light.

The specimen is a part of a human body that contains water, glucose and, depending on the case, ethanol. A finger is an example.

"Absorbances are equal" includes cases of light absorbances matching precisely, cases of absorbances mutually being within the range of measurement error, cases of absorbance values being close to each other to a degree that they are treated as substantially being equal in consideration of measurement accuracy and fluctuations in absorbance at other wavelengths and cases that are separately defined in the present description.

This noninvasive alcohol sensor carries out measurement using light of a wavelength at which the absorbance of water and the absorbance of ethanol are equal. At this wavelength, since the overall absorbance of the specimen does not change even if there are fluctuations in the concentration of ethanol, this wavelength serves as a reference for determining the absorbance of glucose with high accuracy. Once the absorbance of glucose is determined with high accuracy, the absorbance of ethanol can also be determined with high accuracy by subtracting the absorbance of glucose.

The second wavelength may be a wavelength at which the absorbance of ethanol demonstrates a local maximum value.

Here, a local maximum value refers to a local maximum value of a measured spectrum (continuous line). Since measured values of absorbance increase at such a wavelength, measurement error can be held to a relatively low level.

The first wavelength may fall within a range of 1181 to 1189 nm. The absorbance of water and the absorbance of ethanol are equal at a wavelength within this range.

The first light-emitting means, the second light-emitting means and the third light-emitting means may each be a single-wavelength laser element or light-emitting diode.

The noninvasive alcohol sensor may further comprise storage means, and the storage means may store a reference intensity of the first light, a reference intensity of the second light and a reference intensity of the third light detected by the light detection means in the case where a specimen is not present, and a calculation means may calculate the concentration of ethanol contained in the specimen based on the respective differences between the reference intensity of the first light, the reference intensity of the second light and the reference intensity of the third light stored in the storage means, and the intensity of the first light, the intensity of the second light and the intensity of the third light detected by the light detection means.

The third wavelength may be a wavelength at which the light absorbance of water and the light absorbance of ethanol are equal, and which is different from the first wavelength.

In addition, a noninvasive alcohol sensor related to this invention is a noninvasive alcohol sensor that measures a concentration of ethanol contained in a specimen, comprising: first light-emitting means for radiating a first light having a first wavelength towards the specimen, second light-emitting means for radiating a second light having a second wavelength towards the specimen, light detection means for detecting a light intensity, storage means for storing a preliminary measurement intensity of the first light and a preliminary measurement intensity of the second light as intensities detected by the light detection means where the concentration of ethanol contained in the specimen is known, and calculation means for calculating the concentration of ethanol contained in the specimen based on the preliminary measurement intensity of the first light and the preliminary measurement intensity of the second light stored in the storage means and the intensity of the first light and the intensity of the second light detected by the light detection means, wherein the first wavelength is a wavelength at which the light absorbance of water and the light absorbance of ethanol are equal.

Here, preliminary measurement intensity refers to a light intensity measured prior to the time a user desires to measure actual alcohol concentration in a state in which alcohol concentration is known in advance, such as a state in which alcohol has not been consumed and alcohol concentration can be considered to be zero.

Moreover, a noninvasive alcohol sensor related to this invention is a noninvasive alcohol sensor that measures a concentration of ethanol contained in a specimen, comprising: light-emitting means for radiating, towards a specimen, light of three or more wavelengths including a wavelength at which the light absorbance of water and the light absorbance of ethanol are equal, and light detection means for detecting a light intensity, wherein the concentration of ethanol contained in the specimen is calculated based on the intensity of each light detected by the light detection means.

EFFECT OF THE INVENTION

According to this invention, since a noninvasive alcohol sensor carries out measurement by using light of a wavelength at which the light absorbance of water and the light absorbance of ethanol are equal, the light absorbance of glucose can be determined with high accuracy regardless of the concentration of ethanol. Consequently, the effects of the light absorbance of glucose can be removed with high accuracy, thereby making it possible to accurately measure the concentration of ethanol by suppressing error that is dependent on the concentration of glucose.

BRIEF DESCRIPTION OF THE DRAWINGS

FIG. 1 is a drawing schematically showing the construction of an alcohol sensor related to a first embodiment;
FIG. 2 is a graph showing light absorbances demonstrated by four types of solutions at different optical wavelengths, the solutions containing mixtures of water and ethanol at different ratios;
FIG. 3 is a graph showing changes in light absorbance accompanying increases in ethanol; and
FIG. 4 is a drawing schematically showing the construction of an alcohol sensor related to a second embodiment.

EMBODIMENTS FOR CARRYING OUT THE INVENTION

The following provides an explanation of embodiments of the invention based on the attached drawings.

First Embodiment

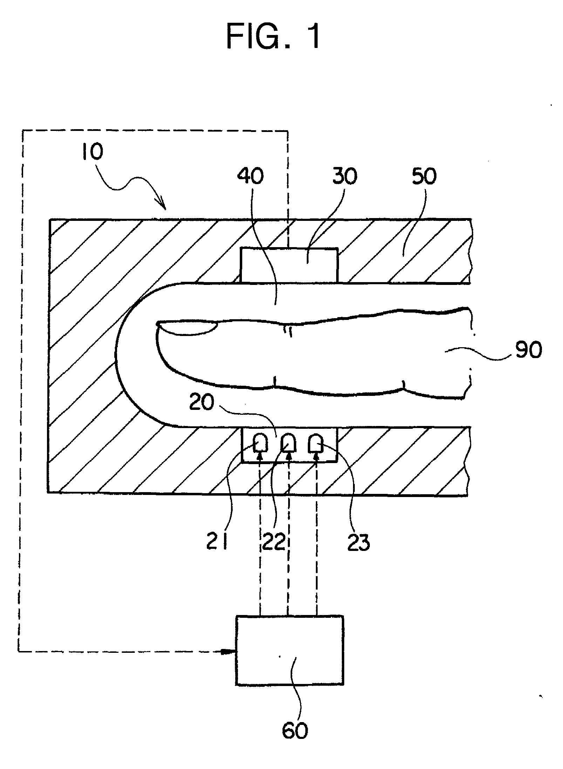

FIG. 1 is a drawing schematically showing the construction of an alcohol sensor 10 related to a first embodiment. The alcohol sensor 10 is a sensor that uses a portion of a human body such as a finger 90 as a specimen, and measures the concentration of ethanol contained in the specimen.

The alcohol sensor 10 carries out measurements noninvasively using light. The alcohol sensor 10 is provided with a light-emitting portion 20 that emits light for measurement. The light-emitting portion 20 includes first light-emitting means 21, second light-emitting means 22 and third light-emitting means 23.

The first light-emitting means 21 is a single-wavelength laser element, and radiates a first light having a first wavelength towards the specimen. Namely, the first light radiated from the first light-emitting means 21 follows a spectral distribution that demonstrates a peak at the first wavelength.

Similarly, the second light-emitting means 22 is a single-wavelength laser element, and radiates a second light having a second wavelength towards the specimen. Moreover, the third light-emitting means 23 is a single-wavelength laser element, and radiates a third light having a third wavelength towards the specimen. The second light and the third light radiated from the second light-emitting means 22 and the third light-emitting means 23 follow spectral distributions that demonstrate peaks at the second and third wavelengths, respectively.

The alcohol sensor 10 is provided with light detection means 30 for detecting the intensities of the first light, the second light and the third light radiated from the first light-emitting means 21, the second light-emitting means 22 and the third light-emitting means 23. The light detection means 30 is, for example, a photodiode, and generates a signal corresponding to a detected light intensity.

A space between the light-emitting portion 20 and the light detection means 30 is a measurement region 40. In the case where the finger 90 is not present in the measurement region 40, the light detection means 30 is able to detect the intensity of light radiated from the light-emitting portion 20 by receiving that light directly. In the case where the finger 90 is present in the measurement region 40, the light detection means 30 is able to receive light that has been radiated from the light-emitting portion 20 and passed through the finger 90 and detect the intensity thereof.

The alcohol sensor 10 is provided with a housing 50 that fixes and supports the light-emitting portion 20 and the light detection means 30. The housing 50 has a shape that blocks light from entering the measurement region 40 from outside the alcohol sensor 10, thereby preventing extraneous light from being detected by the light detection means 30.

In addition, the alcohol sensor 10 is provided with control means 60 that receives a signal from the light detection means 30. The control means 60 contains an electronic circuit such as a microprocessor, and functions as calculation means that calculates the concentration of ethanol contained in the finger 90 based on the intensities of the first, second and third lights detected by the light detection means 30. In addition, the control means 60 contains a memory device such as a semiconductor memory, and functions as storage means for storing the intensities of the first, second and third lights detected by the light detection means 30.

Moreover, the control means 60 is connected to the first light-emitting means 21, the second light-emitting means 22 and the third light-emitting means 23 of the light-emitting portion 20, and controls starting and stopping of the emission of light therefrom.

Furthermore, although not shown in the drawings, the control means 60 is provided with an operating portion that accepts operations from a user, and an output portion that outputs measurement results to a user.

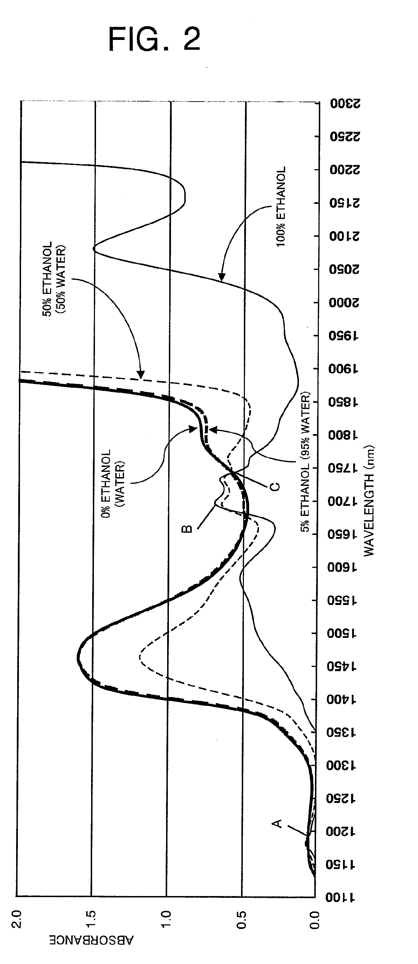

The first light-emitting means 21, the second light-emitting means 22 and the third light-emitting means 23 radiate light of respectively different wavelengths. An explanation of these wavelengths is provided using FIG. 2.

FIG. 2 is a graph showing light absorbances demonstrated by four types of solutions, which contain mixtures of water and ethanol at different ratios, at different optical wavelengths. Light wavelengths are plotted on the horizontal axis in nm units, while logarithms of light absorbance are plotted on the vertical axis. Furthermore, light absorbance Abs is defined as Abs = -log(P2/P1), where P1 is the intensity of light that enters a solution, and P2 is the intensity of light that exits the solution after passing therethrough.

In FIG. 2, the thick solid line indicates the light absorbance of 0% ethanol, namely that of water alone. The thick broken line indicates the light absorbance of 5% ethanol, namely that of a mixture of 5% by weight of ethanol and 95% by weight of water. The narrow broken line indicates the light absorbance of 50% ethanol, namely that of a mixture of 50% by weight ethanol and 50% by weight water. The narrow solid line indicates the light absorbance of 100% ethanol, namely that of ethanol alone.

The first light-emitting means 21 radiates light having a wavelength of 1185 nm as a first wavelength. This corresponds to point A in FIG. 2. At a wavelength of 1185 nm, the absorbances of the four types of solutions are equal. Namely, the absorbance of water and the absorbance of ethanol are equal. Consequently, at this wavelength, even if there is a change in ethanol concentration, the overall absorbance of the specimen does not change, and any fluctuations in absorbance may be thought to be attributable to fluctuations in glucose concentration.

The second light-emitting means 22 radiates light having a wavelength of 1710 nm as a second wavelength. This corresponds to point B in FIG. 2. At a wavelength of 1710 nm, the absorbance of ethanol demonstrates a local maximum value. In addition, this absorbance demonstrates the maximum value within the range of 1100 to 2000 nm. At this wavelength, measurement error can be made to be relatively small since the measured value of absorbance is large. The third light-emitting means 23 radiates light having a wavelength of 1750 nm as a third wavelength. This corresponds to point C in FIG. 2. At a wavelength of 1750 nm, since the absorbances of the four types of solutions are equal in the same manner as point A, the absorbances of water and ethanol are equal. Consequently, at this wavelength, since the overall absorbance of the specimen does not change even if there are fluctuations in the concentration of ethanol, any fluctuations in absorbance may be thought to be attributable to fluctuations in the concentration of glucose.

The following provides an explanation of operation of alcohol sensor 10 having the construction described above. First, before actually measuring the concentration of ethanol using the finger 90 as a specimen, the control means 60 of the alcohol sensor 10 preliminarily stores reference intensities to serve as measurement references for each of the wavelengths at 1185 nm, 1710 nm and 1750 nm.

These reference intensities are determined, for example, in the following manner. First, the control means 60 controls the first light-emitting means 21 to radiate light at 1185 nm in a state in which the finger 90 is not present in the measurement region 40, and acquires the intensity detected by the light detection means 30 in response thereto. The acquired intensity is the reference intensity corresponding to 1185 nm, and is defined as a first reference intensity $I0_{1185}$.

Next, the control means 60 controls the second light-emitting means 22 to radiate light at 1710 nm, and acquires the intensity detected by the light detection means 30 in response thereto. The acquired intensity is the reference intensity corresponding to 1710 nm, and is defined as a second reference intensity $I0_{1710}$.

Moreover, the control means 60 controls the third light-emitting means 23 to radiate light at 1750 nm, and acquires the intensity detected by the light detection means 30 in response thereto. This acquired intensity is the reference intensity corresponding to 1750 nm, and is defined as a third reference intensity $I0_{1750}$.

After having stored the reference intensities in this manner, the control means 60 of the alcohol sensor 10 carries out measurement on an actual specimen.

A user of the alcohol sensor 10 uses the alcohol sensor 10 in order to measure the concentration of alcohol, namely the concentration of ethanol, in the blood following consumption of alcohol, for example. The user first inserts the finger 90 into the housing 50 of the alcohol sensor 10 as shown in FIG. 1, and then positions the finger 90 so as to transverse the measurement region 40, or at least occupy a portion of the measurement region 40. While in this state, the user then operates an operating portion (not shown) of the alcohol sensor 10 and instructs the control means 60 to begin measurement.

The control means 60 then measures ethanol concentration in response to this operation. During measurement, the control means 60 first controls the first light-emitting means 21 to radiate light at 1185 nm and acquires the intensity detected by the light detection means 30 in response thereto. The acquired intensity is the intensity corresponding to 1185 nm and is defined as a first intensity $I_{1185}$.

Next, the control means 60 controls the second light-emitting means 22 to radiate light at 1710 nm and acquires the intensity detected by the light detection means 30 in response thereto. The acquired intensity is the intensity corresponding to 1710 nm and is defined as a second intensity $I_{1710}$.

Moreover, the control means 60 controls the third light-emitting means 23 to radiate light at 1750 nm and acquires the intensity detected by the light detection means 30 in response thereto. The acquired intensity is the intensity corresponding to 1750 nm and is defined as a third intensity $I_{1750}$.

In this manner, the differences in the intensities measured for the finger 90 and the previously described reference intensities correspond to intensities of the light absorbed by the entire finger 90. That is, the finger 90 absorbs light of an intensity corresponding to $\Delta I1 = I0_{1185} - I_{1185}$ for light at a wavelength of 1185 nm, absorbs light of an intensity corresponding to $\Delta I2 = I0_{1710} - I_{1710}$ for light at a wavelength of 1710 nm, and absorbs light of an intensity corresponding to $\Delta I3 = I0_{1750} - I_{1750}$ for light at a wavelength of 1750 nm.

Here, since the finger 90 can be treated as a mixture of water, ethanol and glucose, the following equations (1) to (3) hold with respect to these concentrations.

$$\Delta I1 = I0_{1185} - I_{1185}$$

$$= (C_W + C_E)\Delta d\varepsilon_{W1} + C_G \Delta d\varepsilon_{G1} \qquad (1)$$

$$\Delta I2 = IO_{1710} - I_{1710}$$

$$= C_W \Delta d\varepsilon_{W2} + C_E \Delta d\varepsilon_{E2} + C_G \Delta d\varepsilon_{G2} \qquad (2)$$

$$\Delta I3 = IO_{1750} - I_{1750}$$

$$= (C_W + C_E) \Delta d\varepsilon_{W3} + C_G \Delta d\varepsilon_{G3} \qquad (3)$$

Here, $C_W$, $C_E$ and $C_G$ respectively represent the mol concentration of water, ethanol and glucose contained in the finger 90, and are unknown quantities in equations (1) to (3). Note that changes in molecular volume are treated as indistinguishable from mol concentration in equations (1) to (3). $\Delta d$ represents the optical path length when light passes through the finger 90.

$\varepsilon_{W1}$ and $\varepsilon_{G1}$ in equation (1) represent the absorption coefficients of water and glucose at 1185 nm. Note that, although equation (1) does not include any variable representing the absorption coefficient of ethanol, since the absorbance of ethanol is equal to the absorbance of water at 1185 nm as previously described, the absorption coefficient of ethanol can be treated as being equal to the absorption coefficient $\varepsilon_{W1}$ of water. That is, at this wavelength, since there are no fluctuations in the overall absorbance of the specimen even if there are fluctuations in the concentration $C_E$ of ethanol, provided there are no fluctuations in the sum of the concentration $C_W$ of water and the concentration $C_E$ of ethanol, $\Delta I1$ serves as a reference for determining the concentration $C_G$ of glucose with high accuracy.

$\varepsilon_{W2}$, $\varepsilon_{E2}$ and $\varepsilon_{G2}$ of equation (2) represent the absorption coefficients of water, ethanol and glucose at 1710 nm.

$\varepsilon_{W3}$ and $\varepsilon_{G3}$ of equation (3) represent the absorption coefficients of water and glucose at 1750 nm. Note that, since the absorbance of ethanol is equal to the absorbance of water at 1750 nm as previously described, the absorption coefficient of ethanol can be treated as being equal to the absorption coefficient of water in the same manner as equation (1), and $\Delta I3$ serves a reference for determining the concentration $C_G$ of glucose with high accuracy.

Furthermore, $\Delta d$, $\varepsilon_{W1}$, $\varepsilon_{G1}$, $\varepsilon_{W2}$, $\varepsilon_{E2}$, $\varepsilon_{G2}$, $\varepsilon_{W3}$ and $\varepsilon_{G3}$ are values that can be determined in advance through experimentation and the like by a person with ordinary skill in the art, and are stored as constants in the control means 60.

The control means 60 calculates $C_W$, $C_E$ and $C_G$ by solving the simultaneous equations consisting of equations (1) to (3). Here, the method used to solve the simultaneous equations may be accomplished in any manner, and a suitable algorithm can be determined by a person with ordinary skill in the art. For example, $C_G$ can first be determined along with $C_W + C_E$ from equations (1) and (3), and these can be then be used to determine $C_W$ and $C_E$ from equation (2). Here, since the glucose concentration $C_G$ can be determined with high accuracy according to equations (1) and (3) as previously described, the concentration of water $C_W$ and the concentration of ethanol $C_E$ can also be determined with high accuracy in the same manner.

Subsequently, the control means 60 outputs the calculated value of $C_E$, namely the concentration of ethanol, to an output portion (not shown) and thereby presents the value to the user. In this manner, the user is able to determine the concentration of ethanol contained in the finger 90, or in other words, the concentration of alcohol in the blood.

In this manner, since the alcohol sensor 10 related to the first embodiment carries out measurements by using light having a wavelength of 1185 nm, at which the absorbance of water and the absorbance of ethanol are equal, the absorbance of glucose can be determined with high accuracy regardless of the concentration of ethanol. Consequently, effects attributable to the absorbance of glucose can be removed with high accuracy, and ethanol concentration can be measured with high accuracy by suppressing error dependent upon the concentration of glucose.

In addition, since the light detection means 30 of the alcohol sensor 10 is only required to detect intensity regardless of light wavelength, an inexpensive configuration can be realized without requiring an expensive spectrometer.

In addition, since the alcohol sensor 10 is only required to detect the intensities at three wavelengths, it is not necessary to repeat measurements over a wide range, thereby making it possible to carry out measurements using a simple configuration and in a short period of time.

Although the first wavelength in the first embodiment is 1185 nm, a different wavelength may be used provided it is a wavelength at which the absorbance of water and the absorbance of ethanol are equal.

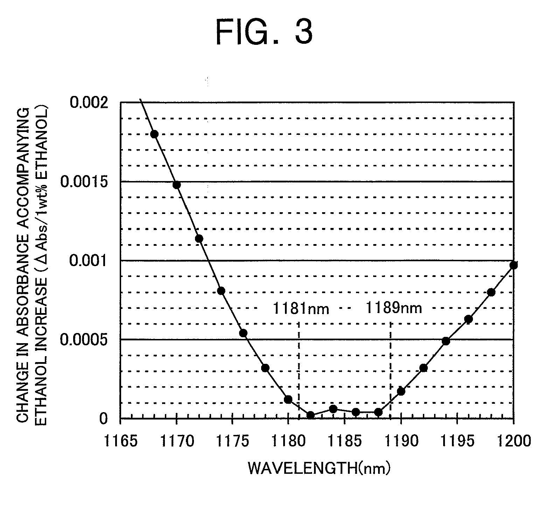

FIG. 3 is a graph showing changes in absorbance accompanying changes in ethanol concentration at a wavelength in the vicinity of 1185 nm. At a wavelength of 1170 nm, for example, absorbance increases only by about 0.0015 when ethanol concentration increases by 1% by weight.

According to the graph of FIG. 3, since the amount of change within the range of 1181 to 1189 nm is substantially zero, and the values within this range are extremely small as compared with those outside this range, the absorbance of water and the absorbance of ethanol can be said to be equal. Thus, if the first wavelength falls within the range of 1181 to 1189 nm, measurement accuracy comparable to the first embodiment can be obtained.

In addition, although the second wavelength in the first embodiment is 1710 nm, this may be another wavelength provided it is a wavelength at which the absorbance of ethanol demonstrates a local maximum value. For example, as shown in FIG. 2, the second wavelength may be 1580 nm since the absorbance of ethanol also demonstrates a local maximum value at 1580 nm. Moreover, the second wavelength may also be 2250 nm since it is known that the absorbance of ethanol also demonstrates a local maximum value at a wavelength of 2250 nm. Moreover, the wavelength may also be wavelength at which the absorbance of ethanol does not demonstrate any local maximum value provided it is a wavelength at which error does not occur that exceeds an allowable limit of final measurement accuracy for the ethanol concentration. In addition, although the third wavelength in the first embodiment is 1750 nm, this may also be a different wavelength provided it is a wavelength at which the absorbance of water and the absorbance of ethanol are equal.

In the first embodiment, the first light-emitting means 21, the second light-emitting means 22 and the third light-emitting means 23 are single-wavelength laser elements. As a variation thereof, other light-emitting means may be used provided they are capable of radiating light having a specific wavelength, and light-emitting diodes, for example, may also be used. Single-wavelength laser element (s) and light-emitting diode(s) may also be used in combination. In addition, another detection means may be used for the light detection means 30 provided it is able to detect light intensity.

Moreover, the light-emitting portion 20 may also be provided with variable wavelength light-emitting means that radiates light at multiple wavelengths.

An example of this variable wavelength light-emitting means that can be used is that which radiates light having a wavelength of 1185 nm, at which the absorbance of water and the absorbance of ethanol are equal, as a first wavelength, light having a wavelength of 1710 nm as a second wavelength, and light having a wavelength of 1750 nm as a third wavelength. Here, the wavelength of the radiated light as well as the starting and stopping of light emission can be controlled. For example, the control means 60 may control selection or switching of wavelength and starting and stopping of light emission at each wavelength.

In addition, although the number of wavelengths able to be radiated may be three as previously described, four or more wavelengths can also be used. For example, the alcohol sensor 10 may be configured to radiate light of desired wavelengths by selecting or adjusting the wavelength used corresponding to a wavelength variably set in the control means 60. Note that the size of light-emitting elements can be reduced even in the case of radiating light at multiple wavelengths in this manner. Such light-emitting elements can be reduced in size in comparison with light-emitting means that radiate light of even larger number of wavelengths or light-emitting means that radiate light of a continuously varying wavelength.

Second Embodiment

A second embodiment reduces the number of wavelengths required for measurement from three to two by employing a construction in which measurements are carried out prior to consumption of alcohol and those results are registered in the first embodiment.

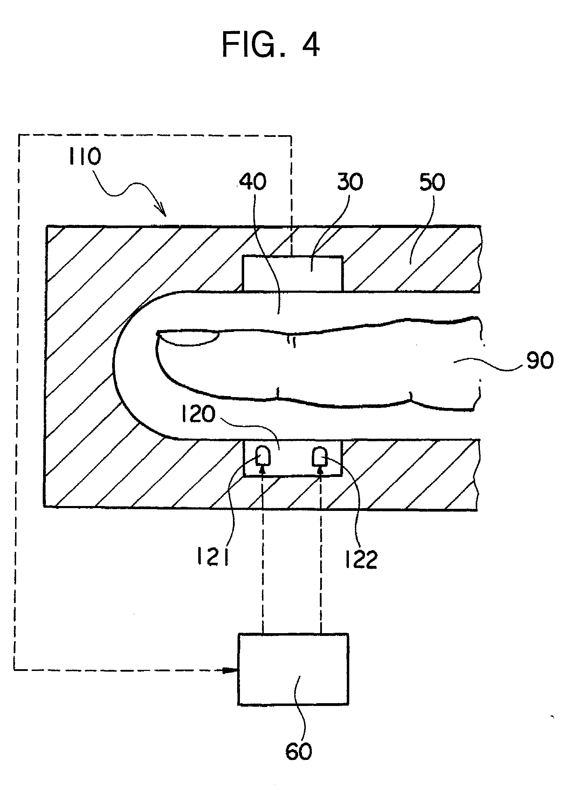

FIG. 4 is a drawing schematically showing the construction of an alcohol sensor 110 related to the second embodiment. The following provides an explanation of differences between this alcohol sensor 110 and the alcohol sensor 10 of FIG. 1.

The alcohol sensor 110 is provided with a light-emitting portion 120 that radiates light for measurement. The light-emitting portion 120 includes first light-emitting means 121 and second light-emitting means 122.

The first light-emitting means 21 radiates a first light having a first wavelength of 1185 nm towards the finger 90 serving as a specimen. This wavelength is a wavelength at which the absorbance of water and the absorbance of ethanol are equal.

The second light-emitting means 22 radiates a second light having a second wavelength of 1710 nm towards the finger 90 serving as a specimen. This wavelength is a wavelength at which the absorbance of ethanol demonstrates a local maximum value.

The light detection means 30 detects the intensities of the first light and the second light.

The alcohol sensor 110 stores the first reference intensity $I0_{1185}$ corresponding to a wavelength of 1185 nm, and the second reference intensity $I0_{1710}$ corresponding to wavelength of 1710 nm in the same manner as the first embodiment.

The alcohol sensor 110 related to the second embodiment utilizes the fact that the ethanol concentration contained in the finger 90 can be considered to be zero when a user has not consumed alcohol, namely that the ethanol concentration is of a known value. A user instructs the control means 60 to begin preliminary measurement on the finger 90 in the absence of alcohol consumption.

In response to this operation, the control means 60 carries out preliminary measurement of ethanol concentration. During the preliminary measurement, the control means 60 first controls the first light-emitting means 121 to radiate light at 1185 nm, and acquires the intensity detected by the light detection means 30 in response thereto. The control means 60 then stores the acquired intensity as a first preliminary measurement intensity $Ip_{1185}$ that is the preliminary measurement intensity corresponding to a wavelength of 1185 nm.

Next, the control means 60 controls the second light-emitting means 122 to radiate light at 1710 nm, and acquires the intensity detected by the light detection means 30 corresponding thereto. The control means 60 then stores the acquired intensity as a second preliminary measurement intensity $Ip_{1710}$ that is the preliminary measurement intensity corresponding to a wavelength of 1710 nm.

The differences between the preliminary measurement intensities and the reference intensities correspond to the intensities of the light absorbed by the entire finger 90. That is, the finger 90 absorbs light of an intensity corresponding to $\Delta Ip1 = I0_{1185} - Ip_{1185}$ for light at a wavelength of 1185 nm, and absorbs light of an intensity corresponding to $\Delta Ip2 = I0_{1710} - Ip_{1710}$ for light at a wavelength of 1710 nm. Accordingly, the following equations (4) and (5) hold.

$$\Delta Ip1 \quad = I0_{1185} - Ip_{1185}$$

$$= C_W \Delta d\varepsilon_{W1} + C_G{}' \Delta d\varepsilon_{G1} \qquad (4)$$

$$\Delta Ip2 \quad = I0_{1710} - Ip_{1710}$$

$$= C_W \Delta d\varepsilon_{W2} + C_G{}' \Delta \varepsilon_{G2} \qquad (5)$$

Here, $C_W$ and $C_G{}'$ respectively represent the mol concentration of water and glucose.

After the user has carried out the preliminary measurements, the alcohol sensor 110 is used to measure alcohol concentration in the blood, for example after consuming alcohol. This measurement is carried out in the same manner as the first embodiment, and as a result thereof, the alcohol sensor 110 acquires the first intensity $I_{1185}$, which is the intensity corresponding to a wavelength of 1185 nm, and the second intensity $I_{1710}$, which is the intensity corresponding to a wavelength of 1710 nm.

Here, the following equations (6) and (7), which contain the concentration $C_W$ of water, the concentration $C_E$ of ethanol and the concentration $C_G$ of glucose with respect to the intensity of light absorbed by the finger 90, hold.

$$\Delta I1 = I0_{1185} - I_{1185}$$

$$= (C_W + C_E)\Delta d\varepsilon_{W1} + C_G \Delta d\varepsilon_{G1} \qquad (6)$$

$$\Delta I2 = I0_{1710} - I_{1710}$$

$$= C_W \Delta d\varepsilon_{W2} + C_E \Delta d\varepsilon_{E2} + C_G \Delta d\varepsilon_{G2} \qquad (7)$$

Furthermore, since the absorbance of ethanol and the absorbance of water are equal at a wavelength of 1185 nm as previously described, and the overall absorbance of the specimen does not change even if there are fluctuations in the concentration $C_E$ of ethanol, $\Delta I1$ serves as a reference for determining the concentration $C_G$ of glucose with high accuracy.

The control means 60 calculates $C_W$, $C_E$, $C_G$ and $C_G{}'$ by solving the simultaneous equations consisting of equations (4) to (7). Here, the method used to solve the simultaneous equations may be accomplished in any manner, and a suitable algorithm can be determined by a person with ordinary skill in the art. For example, $C_W$ and $C_G{}'$ can first be determined from equations (4) and (5), and $C_E$ and $C_G$ can then be determined from the determined $C_W$ and equations (6) and (7).

In this manner, since the alcohol sensor 110 related to the second embodiment carries out measurements by using light having a wavelength of 1185 nm, at which the absorbance of water and the absorbance of ethanol are equal, in the same manner as the first embodiment, the absorbance of glucose can be determined with high accuracy regardless of the concentration of ethanol. Consequently, effects attributable to the absorbance of glucose can be removed with high accuracy, and ethanol concentration can be measured with high accuracy by suppressing error dependent upon the concentration of glucose.

In addition, differing from the first embodiment, since only two light-emitting means are required, the construction can be made even simpler.

Furthermore, the same variations can be made to the second embodiment as those made for the first embodiment.

## Claims

1. A noninvasive alcohol sensor that measures a concentration of ethanol contained in a specimen, comprising:

first light-emitting means for radiating a first light having a first wavelength towards the specimen;
second light-emitting means for radiating a second light having a second wavelength towards the specimen;
third light-emitting means for radiating a third light having a third wavelength towards the specimen;
light detection means for detecting light intensity; and
calculation means for calculating the concentration of ethanol contained in the specimen based on intensity of the first light, intensity of the second light and intensity of the third light detected by the light detection means, wherein
the first wavelength is a wavelength at which the light absorbance of water and the light absorbance of ethanol are equal.

2. The noninvasive alcohol sensor according to claim 1, wherein the second wavelength is a wavelength at which the light absorbance of ethanol demonstrates a local maximum value.

3. The noninvasive alcohol sensor according to claim 1, wherein the first wavelength falls within a range of 1181 to 1189 nm.

4. The noninvasive alcohol sensor according to claim 1, wherein the first light-emitting means, the second light-emitting means and the third light-emitting means are each a single-wavelength laser element or a light-emitting diode.

5. The noninvasive alcohol sensor according to claim 1, wherein the noninvasive alcohol sensor further comprises storage means,
the storage means stores a reference intensity of the first light, a reference intensity of the second light and a reference intensity of the third light detected by the light detection means in the case where a specimen is not present, and
the calculation means calculates the concentration of ethanol contained in the specimen based on the respective differences between the reference intensity of the first light, the reference intensity of the second light and the reference intensity of the third light stored in the storage means, and the intensity of the first light, the intensity of the second light and the intensity of the third light detected by the light detection means.

6. The noninvasive alcohol sensor according to claim 1, wherein the third wavelength is a wavelength at which the light absorbance of water and the light absorbance of ethanol are equal, and which is different from the first wavelength.

7. A noninvasive alcohol sensor that measures a concentration of alcohol contained in a specimen, comprising:

first light-emitting means for radiating a first light having a first wavelength towards the specimen;
second light-emitting means for radiating a second light having a second wavelength towards the specimen;
light detection means for detecting a light intensity;
storage means for storing a preliminary measurement intensity of the first light and a preliminary measurement intensity of the second light as intensities detected by the light detection means where the concentration of ethanol contained in the specimen is known; and
calculation means for calculating the concentration of ethanol contained in the specimen based on the preliminary measurement intensity of the first light and the preliminary measurement intensity of the second light stored in the storage means and the intensity of the first light and the intensity of the second light detected by the light detection means, wherein
the first wavelength is a wavelength at which the light absorbance of water and the light absorbance of ethanol are equal.

8. A noninvasive alcohol sensor that measures a concentration of ethanol contained in a specimen, comprising:

light-emitting means for radiating, towards a specimen, light of three or more wavelengths including a wavelength at which the light absorbance of water and the light absorbance of ethanol are equal;
light detection means for detecting light intensity; and
calculation means for calculating the concentration of ethanol contained in the specimen based on intensity of each light detected by the light detection means.

# FIG. 1

# FIG. 2

# FIG. 3

# FIG. 4

**INTERNATIONAL SEARCH REPORT**

| International application No. |
| --- |
| PCT/JP2009/061315 |

A.  CLASSIFICATION OF SUBJECT MATTER
*A61B5/1455(2006.01)i, G01N21/35(2006.01)i*

According to International Patent Classification (IPC) or to both national classification and IPC

B.   FIELDS SEARCHED

Minimum documentation searched (classification system followed by classification symbols)
A61B5/1455, G01N21/35

Documentation searched other than minimum documentation to the extent that such documents are included in the fields searched

| | | | |
| --- | --- | --- | --- |
| Jitsuyo Shinan Koho | 1922-1996 | Jitsuyo Shinan Toroku Koho | 1996-2009 |
| Kokai Jitsuyo Shinan Koho | 1971-2009 | Toroku Jitsuyo Shinan Koho | 1994-2009 |

Electronic data base consulted during the international search (name of data base and, where practicable, search terms used)

C.   DOCUMENTS CONSIDERED TO BE RELEVANT

| Category* | Citation of document, with indication, where appropriate, of the relevant passages | Relevant to claim No. |
| --- | --- | --- |
| A | JP 2004-248820 A  (Citizen Watch Co., Ltd.),<br>09 September, 2004 (09.09.04),<br>Full text; all drawings<br>(Family: none) | 1-8 |
| A | JP 2008-86724 A  (Koichi YAMANOGAMI),<br>17 April, 2008 (17.04.08),<br>Full text; all drawings<br>(Family: none) | 1-8 |

| [X]  Further documents are listed in the continuation of Box C. | [ ]  See patent family annex. |
| --- | --- |

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered   to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>    10 July, 2009 (10.07.09) | Date of mailing of the international search report<br>    21 July, 2009 (21.07.09) |
| --- | --- |
| Name and mailing address of the ISA/<br>    Japanese Patent Office | Authorized officer |
| Facsimile No. | Telephone No. |

Form PCT/ISA/210 (second sheet) (April 2007)

<table>
<tr><td colspan="2">**INTERNATIONAL SEARCH REPORT**</td><td>International application No.<br>PCT/JP2009/061315</td></tr>
</table>

C (Continuation). DOCUMENTS CONSIDERED TO BE RELEVANT

| Category* | Citation of document, with indication, where appropriate, of the relevant passages | Relevant to claim No. |
|---|---|---|
| A | JP 2007-509718 A (Nellcor Puritan Bennett Inc.),<br>19 April, 2007 (19.04.07),<br>Full text; all drawings<br>& JP 2004-527292 A     & JP 2009-515664 A<br>& US 2004/0230106 A1    & US 2003/0220548 A1<br>& US 6591122 B2         & US 2006/0084864 A1<br>& US 2006/0253016 A1    & US 2007/0129614 A1<br>& US 2006/0020181 A1    & EP 1701653 A<br>& EP 1367938 A          & EP 1948012 A | 1-8 |
| A | US 6615064 B1 (Thomas K. Aldrich),<br>02 September, 2003 (02.09.03),<br>Full text; all drawings<br>& US 6064898 A          & WO 2000/016688 A1<br>& AU 6257599 A | 1-8 |
| A | JP 10-501995 A (Optiscan, Inc.),<br>24 February, 1998 (24.02.98),<br>Full text; all drawings<br>& JP 8-505798 A        & JP 10-510180 A<br>& JP 3590409 B         & US 5515847 A<br>& US 5313941 A        & US 5615672 A<br>& US 5313941 A        & EP 763999 A<br>& EP 682494 A         & EP 901339 A<br>& WO 1995/031930 A1    & WO 1994/016614 A1<br>& WO 1996/017546 A1    & DE 69430366 D<br>& DE 69430366 T | 1-8 |

Form PCT/ISA/210 (continuation of second sheet) (April 2007)

**EP 2 292 145 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2004248820 A